(19) Europäisches Patentamt
European Patent Office
Office européen des brevets

(11) Publication number: **0 116 406 B1**

(12) # EUROPEAN PATENT SPECIFICATION

(45) Date of publication of patent specification: **03.04.91**

(51) Int. Cl.[5]: **A61K 7/32**, A61K 7/021

(21) Application number: **84300135.5**

(22) Date of filing: **10.01.84**

(54) **Colored anhydrous hydrophobic compositions for cosmetic, medicinal and like purposes, and their production.**

(30) Priority: **11.01.83 US 457137**

(43) Date of publication of application:
**22.08.84 Bulletin 84/34**

(45) Publication of the grant of the patent:
**03.04.91 Bulletin 91/14**

(84) Designated Contracting States:
**DE FR GB IT**

(56) References cited:
**GB-A- 2 107 186**
**US-A- 4 126 679**

**PATENTS ABSTRACT OF JAPAN, vol. 1, no. 97, (C-78)[2046], 30th August 1977; JP - A - 53 72 833 (TAKEDA YAKUHIN KOGYO K.K.) 28.06.1978**

(73) Proprietor: **THE MENNEN COMPANY**
**Hanover Avenue**
**Morristown New Jersey 07960(US)**

(72) Inventor: **Kasat, Radhakrishna B.**
**65 Oxford Place**
**Belle Mead New Jersey 08502(US)**

(74) Representative: **Hale, Stephen Geoffrey et al**
**J.Y. & G.W. Johnson Furnival House 14/18**
**High Holborn**
**London WC1V 6DE(GB)**

**EP 0 116 406 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to colored anhydrous, hydrophobic compositions. It is particularly concerned with colored cosmetic-drug compositions, especially antiperspirant compositions having a physiologically acceptable water-soluble antiperspirant compound dispersed in a matrix or carrier which is essentially anhydrous and hydrophobic.

There are many useful compositions whose formulation is essentially anhydrous or hydrophobic. These include waxes and gels for cleaning and polishing automobiles, boats and furniture, cosmetic and cosmetic-drug products including certain types of cleansing creams, certain sun-screen creams and, most importantly, anhydrous antiperspirant compositions for stick, role-on or cream applicators. Many of these compositions, particularly the cosmetic-drug products as formulated, have a rather unattractive white or milky color and their appearance is believed to have an adverse effect on consumer acceptance.

The terms "drug" and "cosmetic" are used herein in the sense of the United States Food and Drug law, 21 U.S.C. 321 (9) (1) and 321(i), respectively. The term "cosmetic-drug" though not officially defined, is generally recognized as embracing compositions which are both cosmetics and drugs as these terms are used in Title 21, United States Code. Antiperspirants are "cosmetic-drugs" as are certain cleansing creams.

To counter the unattractive appearance referred to attempts have been made to introduce color into the compositions. Since the compositions are essentially anhydrous and hydrophobic most such attempts have used oil soluble dyes or inorganic pigments. It has been found that the use of such materials, while successful in coloring the products, is impractical, particularly in cosmetic and cosmetic-drug products, because such colors cause staining of the user's clothing. On the other hand, when water-soluble dyes are introduced as such into the anhydrous matrix, they do not effectively color the matrix, but tend to agglomerate or ball-up.

Antiperspirant compositions of necessity contain an effective antiperspirant agent. The number and composition of these agents is controlled in the United States by the Food and Drug Administration and the number of proposed agents is currently rather limited. They are in general aluminum or aluminum-zirconium salts or complexes. See 47 Federal Register 36504, 20 August 1982. They include aluminum chloride, aluminum chlorohydrate, aluminum chlorohydrex, aluminum chlorohydrex PEG, aluminum chlorohydrex PG, aluminum dichlorohydrate, aluminum dichlorohydrex PEG, aluminum dichlorohydrex PG, aluminum sesquichlorohydrate, aluminum sesquichlorohydrex PEG, aluminum sesquichlorohydrex PG, aluminum sulfate, aluminum zirconium octachlorohydrate, aluminum zirconium octachlorohydrex GLY, aluminum zirconium pentachlorohydrate, aluminum zirconium pentachlorohydrex GLY, aluminum zirconium tetrachlorohydrate, aluminum zirconium trichlorohydrate, aluminum zirconium tetrachlorohydrate GLY, aluminum zirconium trichlorohydrate GLY. See CTFA Cosmetic Ingredient Dictionary, Third Edition, pp. 10-13, 279.

It has now been found that if a water-soluble dyestuff is added to an aqueous solution of a water soluble solid, such as the physiologically acceptable water-soluble antiperspirant agents referred to above and the water removed to give a colored product, this product can be comminuted to form a fine powder which can then be introduced into the anhydrous hydrophobic matrix to give attractive, stable and even color. The invention therefore in one aspect includes a coloured composition comprising an anhydrous, hydrophobic matrix having the consistency of a wax, gel, cream or oil and, dispersed therein, a water-soluble solid with which is intimately associated a water-soluble dyestuff.

The invention is particularly applicable to cosmetic-drug compositions and in a preferred embodiment therefore comprises a colored, cosmetic-drug composition comprising an anhydrous, hydrophobic matrix having the consistency of a wax, gel, cream or oil and, dispersed therein, a physiologically acceptable, water-soluble solid with which is intimately associated a physiologically acceptable, water-soluble dyestuff.

The term "physiologically acceptable" is used to mean a material which may be used in cosmetic-drug preparations for external use without violation of the appropriate regulations of the United States Food and Drug Administration.

The invention is particularly applicable to antisperspirant compositions and in another and preferred aspect it includes a colored antiperspirant composition comprising an anhydrous hydrophobic matrix and, dispersed therein, a physiologically acceptable water-soluble antiperspirant having intimately associated therewith a physiologically acceptable, water-soluble dyestuff.

The invention further includes a method for coloring a hydrophobic, anhydrous composition having the consistency of a wax, gel, cream or oil which comprises mixing an aqueous solution of a water-soluble salt with a water-soluble dyestuff, removing water from the mixture to give a colored crystalline product, reducing the crystalline product to powder and mixing the powder with the other ingredients of the composition.

It also includes a method of preparing a coloured antiperspirant composition according to the invention

comprising mixing an aqueous solution of the antiperspirant with a water-soluble physiologically acceptable dyestuff, removing water from the resulting mixture to give a dry product, reducing the product to powder form and mixing the powder with matrix ingredient(s).

The nature of the composition to which the colored inorganic salt is added may vary widely and although the invention will be described chiefly in connection with antiperspirant compositions it will be understood that this description is illustrative only and that the invention is applicable wherever it is desired to color an anhydrous, hydrophobic material.

The antiperspirant compositions which may be colored in accordance with the invention will include any of those known compositions which include a physiologically acceptable active antiperspirant and a hydrophobic, anhydrous matrix. These will include for example, the thioxotropic creams disclosed in United States Patent 4,083,956 (Shelton) which are composed of a liquid organic emollient material, an inorganic clay suspending and thickening agent, a gel promoting agent and the active astringent antiperspirant. Also included are the stick compositions defined in United States Patent 4,049,792 (Elsnau) which include a water-insoluble wax, a water-insoluble emollient and an active aluminum or zirconium astringent antiperspirant. Further, the composit ions include those disclosed in United States Patent 4,126,679 (Davy et al) which include a volatile silicone oil, a long chain fatty alcohol and the inorganic aluminum or zirconium astringent. It will be understood that the amount of dyestuff incorporated in the compositions is very small indeed, usually not exceeding about 0.1% on the weight of total composition. Thus the nature of the composition, insofar as its antiperspirant or other properties are concerned, remains unaltered, only the appearance being affected.

In general, preferred stick antiperspirant compositions according to the invention will include:

| | Percent By Weight |
|---|---|
| physiologically acceptable antiperspirant | 12 to 25 |
| volatile silicone oil or emollient | 35 to 65 |
| fatty C14 - C30 alcohol, straight chain | 10 to 30 |
| water-soluble, physiologically acceptable dyestuff | 0.0002 to 0.1 |

The fatty alcohol may be replaced in part by other waxy materials, for example by hydrogenated castor oil.

Preferred roll-on compositions according to the invention will in general, comprise:

| | Percent By Weight |
|---|---|
| volatile silicone oil | 60 - 80 |
| gelling agent | 2 - 25 |
| physiologically acceptable antiperspirant | 12 - 25 |
| water-soluble physiologically acceptable dyestuff | 0.0002 - 0.1 |

The volatile silicone oils which are used in the preferred products according to the invention are in general polydimethylcyclosiloxanes having 3 to 6 silicon atoms and linear polydimethyl siloxanes having a viscosity of not more than about l0 centistokes at 25° C.

Such products are commercially available from several manufacturers and include the tetrameric and pentameric dimethyl cyclosiloxanes having the structures:

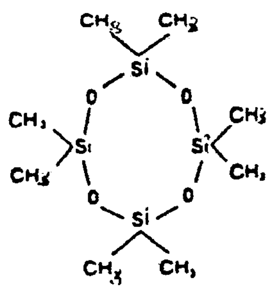 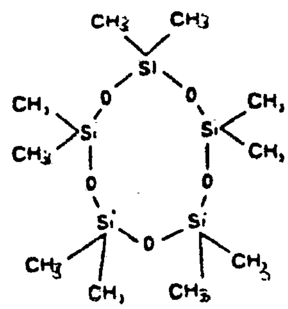

as well as linear compounds such as hexamethyl disiloxane

$(H_3C)_3$ Si-0-Si$(CH_3)_3$

The physiologically acceptable antiperspirant includes any of those which have been approved by the United States Food and Drug Administration. As noted above, these have been identified in the Federal Register for August 20, 1982 and in the CTFA Dictionary and include various aluminum and aluminum zirconium salts. Obviously, other water-soluble astringent salts or complexes can also be employed if desired and if FDA approval is not a consideration.

The dyestuffs which can be employed should be water-soluble and physiologically acceptable. Such dyestuffs are listed in the CTFA Cosmetic Ingredient Dictionary and include the following:

| Name | Color Index Numbers |
|---|---|
| D. & C. Blue No. 4 | CI 42090 ($NH_4$ Salt) |
| D. & C. Brown No. 1 | CI 20170 |
| D. & C. Green No. 5 | CI 61570 |
| D. & C. Green No. 8 | CI 59040 |
| D. & C. Orange No. 4 | CI 15510 |
| D. & C. Orange No. 11 | CI 45425 |
| D. & C. Red No. 6 | CI 15850 |
| D. & C. Red No. 8 | CI 15585 |
| D. & C. Red No. 22 | CI 45 380 (Na Salt) |
| D. & C. Red No. 28 | CI 45 410 (Na Salt) |
| D. & C. Red No. 31 | CI 15800 (Ca Salt) |
| D. & C. Red No. 33 | CI 17200 |

| | |
|---|---|
| D. & C. Yellow No. 8 | CI 45350 (Na Salt) |
| D. & C. Yellow No. 10 | CI 47005 |
| Ext. D. & C. Violet No. 2 | CI 60730 |
| Ext. D. & C. Yellow No. 7 | CI 10316 |
| F.D. & C. Blue No. 1 | CI 42090 |
| F.D. & C. Green No. 3 | CI 42053 |
| F.D. & C. Red No. 3 | CI 45430 |
| F.D. & C. Red No. 40 | CI 16035 |
| F.D. & C. Yellow No. 5 | CI 19140 |
| F.D. & C. Yellow No. 6 | CI 15985 |

The fatty alcohol used in stick formulas according to the invention may be any straight chain fatty alcohol of fourteen to thirty carbon atoms, inclusive. Stearyl alcohol is preferred.

Other materials may be added to the formulation such as fillers like talc, starch or modified starches.

In roll-on formulations according to the invention the suspending or the gelling agent will normally be a clay such as bentonite, hectorite, montmorillonite or a modified clay such as stearalkonium hectorite, sold under the name "Bentone 27" by NL Chemicals, and quarternium - 18 hectorite sold under the name "Bentone 38" by NL Chemicals.

It will be understood that the compositions referred to are all entirely conventional, except for the manner in which they are colored, that is except for the use of a water-soluble dye introduced in intimate association with a water-soluble solid.

In preparing compositions according to the invention, the dyestuff may first be mixed with the solid. Many astringent antiperspirant salts are supplied commercially as aqueous solutions. The dyestuff may be added to such commercial solutions as a solid or as an aqueous solution.

In those cases where the solid is procured as a solid, prior to adding the dye the solid is first put into an aqueous solution of any convenient concentration, depending on the solubility of the solid and, possibly, on the effect that the solid concentration has on the solubility of the dyestuff. It is also possible to mix the dye and solid dry and then add water to form a solution.

The proportion of dyestuff added may be calculated so that when the colored solid is incorporated in the final composition in the amount desired, the dyestuff concentration will be sufficient to give the desired color level. Ordinarily this will amount to between about 0.0002% and about 0.1% on the weight of the end composition.

Frequently it is convenient to make a color concentrate by adding a much larger amount of dyestuff to the solid, to form a mixture containing up to say 30% dye on weight of dry colored solid. After water removal and comminution the colored powder can be mixed with an appropriate quantity of reduced uncolored solid and the mixture added to the final composition. Alternatively the colored and uncolored solid may be added to the other ingredients separately.

Certain dyestuffs in the group described above will not wholly dissolve in the solution with the solid and may form cloudy dispersions or even dark precipitates with certain solids. Such combinations are not preferred. They are useable, however, since after water removal the crystalline product is still colored, with the dye precipitate forming dark spots within the crystals. Upon reducing the crystals to powder these dye deposits become mixed with the colored powder and for the most part are not readily apparent in the ultimate product.

After the dyestuff and solid have been mixed in solution, water is removed. Normally this is done by drying in air at elevated temperature. Generally speaking the temperature is not critical and temperatures of say 40-105 °C have been used. Some solids and some dyestuffs are more temperature sensitive than others and the drying temperature for any particular combination must be selected having regard to effect of temperature on the color stability of the combinations.

Instead of drying by simple heating, the colored solid may be spray dried in any conventional

apparatus.

Certain salts, notably $AlCl_3$ are difficult to dry because of their deliquescent nature and for that reason are not preferred in the compositions of the invention.

Following drying, the colored solid, now in dry crystalline form, is reduced in size to 0.5-100 microns, preferably below about 40 microns, with at least 85% being preferably below 10 microns. There is really no lower limit for particle size, but usually it is not economical to grind to an average particle size less than 4 microns. Grinding can be carried out in any conventional apparatus such as a micropulverizer.

With the colored solid reduced to a fine powder, the colored composition can then be formed according to whatever technique is optimum for the particular composition involved. In the case of solid antiperspirant sticks, the waxy ingredients are heated to a (moderately) elevated temperature, say 70-80 °C, to melt them, after which the normally liquid components and the solids, including the colored solid, are added, and the mixture put into molds and allowed to cool and harden.

With other kinds of compositions appropriate known techniques may be used.

It will be understood that the final compositions may contain fillers, fragrances, stabilizers and the like, all according to conventional practice.

The invention will be further described with reference to the following specific examples which are given as illustrative only.

## Example 1

Five hundred grams (500g) of a 35% solution of aluminum zirconium tetrachlorohydrex GLY were mixed with 10g of a 1% solution of F.D. & C Blue No. 1. After tray drying at 40.8 °C the resulting blue salt was milled to 85% less than 10 microns particle size. This colored powder (4.5g) was then mixed with 17.5 g of uncolored GLY powder, milled to the same particle size.

The colored antiperspirant powder was then mixed with tetrameric dimethyl cyclosiloxane and other ingredients to form an antiperspirant composition for roll-on application, the total formulation being:

| | Parts By Weight |
|---|---|
| dimethyl cyclosiloxane tètramer | 67.0 |
| bentone/silicone gel | 10.00 |
| colored GLY salt | 22.00 |
| fragrance | 1.00 |
| | 100.00 |

The material had an attractive light blue color which was smooth and homogeneous throughout the composition.

The bentone-silicone gel referred to in Example 1 is a mixture of cyclomethicone, quarterium-18 hectorite and propylene carbonate.

## Example 2

Another composition was made up as in Example 1, using the same colored GLY salt but replacing the tetrameric cyclosiloxane with pentameric dimethyl cyclosiloxane. Equivalent results were obtained.

## Example 3

Four hundred grams (400g) of a 50% aqueous solution of aluminum chlorhydrate were mixed with 10g of a 1.0% aqueous solution of F.D. & C. Blue No. 1. The mixture was dried at 120 °F (49 °C) and ground to 85% less than 10 microns. The following roll-on compositions were then made up, parts by weight:

| | 3A | 3B |
|---|---|---|
| dimethyl cyclosiloxane tetramer | 67.0 | |
| dimethyl cyclosiloxane (pentamer) | | 67.0 |
| bentone-silicone gel (as in Example 1) | 10.0 | 10.0 |
| colored aluminum chlorohydrate | 3.0 | 3.0 |
| uncolored aluminum chlorohydrate | 19.0 | 19.0 |
| Fragrance | 1.0 | 1.0 |
| | 100.00 | 100.00 |

The products were an even delicate blue, equivalent to that of Example 1.

Example 4

Thirty-five grams (35g) of a 35% solution of Rehydrol II, a coordination complex of aluminum chlorohydrate and propylene glycol, termed "aluminum chlorohydrex PG" in CTFA nomenclature, was mixed with 2g of a 1% aqueous solution of F.D. & C. Blue No. 1, dried at 105° F (40.8° C) and ground to 85% less than 10 microns. This was substituted for the colored aluminum chlorohydrate of Example 3A with similar results.

Example 5

Using colored powder made as in Example 4 and a white powder prepared by grinding Rehydrol II, uncolored, to the same particle size, an antiperspirant stick composition was then made up having the composition described below. Conventional procedures were used in that the waxy materials, stearyl alcohol and castor wax were melted and mixed with the silicone oil. The other active ingredients were then added to the liquid materials at a temperature of about 70° C. The resulting mixture was poured into a container and allowed to cool.

| | Parts By Weight |
|---|---|
| Colored Rehydrol II | 3.5 |
| Uncolored Rehydrol II | 18.5 |
| Arlacel 165 | 1.0 |
| Dimethyl cyclosiloxane pentamer | 42.0 |
| Stearyl alcohol | 12.0 |
| Castor wax (m.p. 70°C) | 8.0 |
| Talc | 8.5 |
| Fluid AP | 6.5 |
| | 100.0 |

A homogenous blue stick was obtained.

Fluid AP is polypropylene glycol ether of butyl alcohol having the formula $C_4H_9(OCH(CH_3)CH_2)_nOH$, where n is 14 average. It is made by Union Carbide Corp. Arlacel 165 is a non-ionic emulsifying agent, a mixture of glyceryl stearate and polyethylene glycol ester of stearic acid having the formula $CH_3(CH_2)_{16}CO-(OCH_2CH_2)_nOH$ where n is 100 average. It is made by ICI United States Inc.

Example 6

To 500g of a 35% aqueous solution of aluminum zirconium tetrachlorohydrex GLY was added 10g of a 1% aqueous solution of F.D. & C. Blue No. 1. The colored solution was evaporated to dryness at 105° F (40.8° C) and then pulverized in a micropulverizer to a particle size of 85% less than 10 microns. A stick composition was then formed as in Example 5 using the same formulation but with 4.5 parts of colored GLY powder and 17.5 parts of uncolored GLY powder in place of the Rehydrol II. A homogeneous blue stick was obtained.

Example 7

To 400g of a 50% aqueous solution of aluminum chlorohydrate were added 10g of an aqueous 1% solution of F.D. & C. Blue No. 1. The solution was evaporated to dryness at 105° F(40.8° C) and ground to a particle size such that 85% was less than 10 microns. A stick was then made up in the usual manner, having the following composition:

| | |
|---|---|
| colored aluminum chlorohydrate | 2.2 |

| | |
|---|---|
| uncolored aluminum chlorohydrate | 19.8 |
| arlacel 165 | 1.0 |
| dimethyl cyclosiloxane pentamer | 41.0 |
| stearyl alcohol | 12.0 |
| castor wax (m.p. 70°C) | 8.0 |
| talc | 8.5 |
| fluid AP | 6.5 |
| fragrance | 1.0 |
| | 100.00 |

The stick had a homogeneous pale blue color.

Example 8

To 400g of a 50% aqueous solution of aluminum chlorohydrate were added 10g of a 1% solution of F.D. & C. Blue No. 1. The colored solution was evaporated to dryness at 120°F (49°C) and ground to a particle size less than 100 microns.

A stick was then made up in the usual way having the following composition:

| | Part By Weight |
|---|---|
| dimethyl cyclosiloxane pentamer | 52.5 |
| stearyl alcohol | 24.0 |
| arlacel 165 | 1.0 |
| colored aluminum chlorohydrate | 2.2 |
| uncolored aluminum chlorohydrate | 19.8 |
| fragrance | 0.5 |
| | 100.00 |

The stick was a homogeneous light blue color.

Example 9

Using the procedure described in Example 4, Rehydrol II powder colored with F.D. & C. Blue No. 1 was prepared. Using this powder a stick having the following composition was prepared in the usual manner:

| | Part By Weight |
|---|---|
| colored rehydrol II powder | 3.5 |
| uncolored rehydrol II powder | 18.5 |
| dimethyl cyclosiloxane pentamer | 52.5 |
| stearyl alcohol | 24.0 |
| arlacel 165 | 1.0 |
| fragrance | 0.5 |
| | 100.00 |

The stick was a homogeneous light blue color.

Example 10

To 500g of a 35% aqueous solution of aluminum zirconium tetrachlorohydrex GLY were added 10g of a 1% aqueous solution of F.D. & C. Blue No. 1. After evaporating to dryness at 105° F(40.8° C) the colored salt was ground to less than 100 microns and used to make a stick having the following composition:

| | Part By Weight |
|---|---|
| colored GLY powder | 3.5 |
| uncolored GLY powder | 18.5 |
| arlacel 165 | 1.0 |
| dimethyl cyclosiloxane pentamer | 52.5 |
| stearyl alcohol | 24.0 |
| fragrance | 0.5 |
| | 100.00 |

The stick was a homogenous light blue color.

The precise nature of the association between the water-soluble solid and the dye is not known, though in the case of crystalline salts it is postulated that the dye may be present in the water of crystallization. In any event, it is very closely associated with the solid, beyond the level of a close mechanical mixture. To illustrate this a colored salt was prepared by adding 0.01g of F.D. & C. Blue No. 1 to 99.99g of aluminum chlorohydrate which had previously been ground to 85% less than 10 microns, and the two thoroughly mixed by vigorous shaking in a jar to give what appeared to be a homogeneously colored powder. Another sample was prepared by mixing 99.99g of the powdered aluminum chlorhydrate with 0.01g of the dye with mortar and pestle to a homogenously colored powder. A third sample was prepared by mixing aluminum zirconium tetrachlorohydrex GLY, ground to the same particle size, with the dye, in the same proportions, with mortar and pestle. The dyestuff in all cases was less than 10 microns. The three colored powders were then incorporated in antiperspirant stick compositions of the following formulations:

Example 11

| | Exhibits | | |
|---|---|---|---|
| | 11A | 11B | 11C |
| colored aluminum chlorohydrate antiperspirant with color added by grinding in mortar | 22.0 | | |
| colored aluminum chlorohydrate antiperspirant with color added by shaking in jar | | 22.0 | |
| colored Al/Zr GLY antiperspirant with color added by grinding with mortar | | | 22.0 |
| arlacel 165 | 1.0 | 1.0 | 1.0 |
| dimethyl cyclosiloxane pentamer | 53.0 | 41.0 | 53.0 |
| stearyl alcohol | 24.0 | 12.0 | 24.0 |
| castor wax, m.p. 70°C | | 8.0 | |
| talc | | 8.5 | |
| fluid AP | | 6.5 | |
| fragrance | | 1.0 | |
| | 100.00 | 100.00 | 100.00 |

The stick compositions were prepared in the usual way, by melting the stearyl alcohol at about 75° C adding the other ingredients with stirring, pouring into a container and cooling. Sticks 11A and 11B had a very faint bluish-green cast, scarcely perceptible. Stick 11C appeared white at first view. Only upon careful examination could a few blue lines and areas be distinguished.

Example 12

A number of physiologically acceptable water-soluble dyestuffs were added to solutions of aluminum chlorohydrate and aluminum zirconium tetrachlorohydrex GLY solutions to determine their compatibility. In these tests, 5 5g of a 1% dye solution were added to 200g of a 50% chlorohydrate solution, and 2.5g of a 1% dye solution were added to 100g of a 36% GLY solution. The results were as follows:

| Dyestuff | Aluminum Chlorohydrate | Aluminum Zirconium GLY Complex |
|---|---|---|
| D. & C. Yellow No. 10 | v. slight ppt | v. slight ppt |
| D. & C. Brown No. 1 | v. slight ppt | clear |
| D. & C. Green No. 8 | clear | clear |
| D. & C. Red No. 22 | clear | clear |
| Ext D. & C. Violet No. 2 | clear | clear |
| D. & C. Red No. 33 | clear | clear |
| D. & C. Orange No. 4 | heavy ppt | heavy ppt |
| F. D. & C. Yellow No. 5 | clear | clear |
| F. D. & C. Red No. 3 | v. slight haze | clear |
| F. D. & C. Blue No. 1 | clear | clear |
| F. D. & C. Red No. 4 | slight haze | med. ppt |
| F. D. & C. Yellow No. 6 | clear | med. ppt |
| F. D. & C. Red No. 40 | v. slight haze | med. ppt |
| F. D. & C. Green No. 3 | clear | clear |
| D. & C. Yellow No. 8 | v. slight haze | clear |
| D. & C. Red No. 28 | v. slight haze | clear |

The products from D.& C. Orange No. 4, which showed the heaviest precipitate, were evaporated to dryness at 105°F (40.8°C). When broken into granular particles, the precipitate appeared as small dark spots in the colored crystalline granules.

While the foregoing examples have been directed to antiperspirant compositions, it will be clear that the invention is applicable to various other compositions having a hydrophobic, anhydrous matrix. Moreover, even in cosmetic-drug compositions, such as antiperspirants, the color need not be associated with the active ingredient. Solids having neutral effects, such as sodium sulfate, sodium chloride, urea, sugar, and glycine may be used as the dye carrier. Since the proportion of dyestuff needed to be added to the end product is normally very small and can be added to the carrier in a fairly high proportion, up to say 30%, the proportion of carrier needed to be added to the composition is very small, and hence its effect on the properties of the final product is minimal.

The antiperspirant sticks described above are solid colored sticks. An interesting and attractive variation on these products can be made by pouring into the same container, either sequentially or simultaneously, two or more compositions having different colors, or one which is colored and one which is uncolored. In this way, for example, marbled or variegated sticks can be formed. Compositions according to the invention have the unique advantage in such combinations that the color will not bleed, diffuse or migrate from one composition to an adjacent composition.

## Claims

**1.** A colored composition comprising anhydrous, hydrophobic matrix having the consistency of a wax, gel,

cream or oil and, dispersed therein, a water-soluble solid, characterised in that the composition is colored by means of a water-soluble dyestuff intimately associated with the water-soluble solid.

2. A colored cosmetic-drug composition comprising an anhydrous, hydrophobic matrix having the consistency of a wax, gel, cream or oil and, dispersed therein, a water-soluble physiologically acceptable solid, characterised in that the composition is colored by means of a water-soluble, physiologically acceptable dyestuff intimately associated with the water-soluble physiologically acceptable solid.

3. A colored antiperspirant composition comprising an anhydrous, hydrophobic matrix and, dispersed therein, a physiologically acceptable antiperspirant, characterised in that the composition is colored by a physiologically acceptable, water-soluble dyestuff intimately associated with the physiologically acceptable antiperspirant.

4. A composition as claimed in claim 3, characterised in that the antiperspirant is selected from aluminum and aluminum-zirconium astringent salts.

5. A composition as claimed in claim 4, characterised in that the antiperspirant is selected from aluminum chlorohydrate, aluminum-zirconium tetrachlorohydrate glycine complex and aluminum chlorohydrate propylene glycol complex.

6. A composition as claimed in any of claims 3 to 5, characterised in that the dyestuff is present in a proportion by weight of from about 0.0002% to about 0.1% on the weight of the composition.

7. A composition as claimed in any of claims 3 to 6, characterised in that it is in the form of a stick comprising a volatile silicone oil and a long chain fatty alcohol, as well as the physiologically acceptable antiperspirant having intimately associated therewith a water-soluble physiologically acceptable dyestuff.

8. A composition as claimed in claim 7, characterised in that the stick further includes a physiologically acceptable wax.

9. A composition as claimed in claim 7 or 8, characterised in that the stick comprises portions having different colors.

10. A composition as claimed in claim 7 or 8, characterised in that it comprises from about 12 to about 25% by weight of the physiologically acceptable antiperspirant containing intimately associated therewith a water-soluble physiologically acceptable dyestuff; from about 35 to about 65% by weight of the volatile silicone oil; and from about 10 to about 30% by weight of a high molecular weight fatty alcohol, the proportion of dyestuff being from about 0.0002 to about 0.1% by weight of the composition.

11. A composition as claimed in claim 10, characterised in that the fatty alcohol is stearyl alcohol.

12. A composition as claimed in any of claims 3 to 6, characterised in that it is suitable for use in a roll-on dispenser and comprises a volatile silicone oil and, dispersed therein, the physiologically acceptable antiperspirant having intimately associated therewith a physiologically acceptable water-soluble dyestuff.

13. A composition as claimed in claim 12, characterised in that it comprises from about 12 to about 25% by weight of the physiologically acceptable antiperspirant, at least a portion of said antiperspirant having a water soluble physiologically acceptable dyestuff intimately associated therewith, from about 2 to about 25% by weight of a gelling agent, and from about 60 to about 80% by weight of the volatile silicone oil, said dyestuff comprising from about 0.0002% to about 0.1% by weight of the composition.

14. An antisperspirant stick comprising an astringent antiperspirant salt dispersed in an anhydrous, hydrophobic matrix, characterised in that the stick has colored portions and portions without color, the astringent salt antiperspirant in the colored portions having intimately associated therewith a physiologically acceptable water-soluble dye-stuff.

15. A method for preparing a colored composition according to claim 1, characterised by the steps of mixing an aqueous solution of a water-soluble solid and a water-soluble dyestuff, removing water from the resulting mixture to give a colored solid, reducing the solid to powder form and mixing the colored powder with the other ingredient(s) of said composition.

16. A method for preparing a colored antiperspirant composition according to claim 3, characterised by the steps of mixing an aqueous solution of the physiologically acceptable antiperspirant with a water-soluble, physiologically acceptable dyestuff, removing water from the resulting mixture to give a dry product, reducing the product to powder form and mixing the powder with matrix ingredient(s).

## Revendications

1. Composition colorée comprenant une matrice hydrophobe anhydre ayant la consistance d'une cire, d'un gel, d'une crème ou d'une huile et dans laquelle est dispersé un solide hydrosoluble, caractérisée en ce que la composition est colorée au moyen d'un colorant hydrosoluble intimement associé avec le solide hydrosoluble.

2. Composition cosmétique-médicamenteuse colorée comprenant une matrice hydrophobe anhydre ayant la consistance d'une cire, d'un gel, d'une crème ou d'une huile et dans laquelle est dispersé un solide hydrosoluble physiologiquement acceptable, caractérisée en ce que la composition est colorée au moyen d'un colorant hydrosoluble physiologiquement acceptable intimement associé avec le solide hydrosoluble physiologiquement acceptable.

3. Composition antitranspirante colorée comprenant une matrice hydrophobe anhydre et dans laquelle est dispersé un antitranspirant physiologiquement acceptable, caractérisée en ce que la composition est colorée au moyen d'un colorant hydrosoluble physiologiquement acceptable intimement associé avec l'antitranspirant physiologiquement acceptable.

4. Composition selon la revendication 3, caractérisée en ce que l'antitranspirant est choisi parmi les sels astringents d'aluminium et d'aluminium-zirconium.

5. Composition selon la revendication 4, caractérisée en ce que l'antitranspirant est choisi parmi le chlorhydrate d'aluminium, le complexe tétrachlorhydrate d'aluminium-zirconium-glycine et le complexe chlorhydrate d'aluminium-propylèneglycol.

6. Composition selon l'une quelconque des revendications 3 à 5, caractérisée en ce que le colorant est présent en une proportion pondérale d'environ 0,0002% à environ 0,1% du poids de la composition.

7. Composition selon l'une quelconque des revendications 3 à 6, caractérisée en ce qu'elle se présente sous la forme d'un bâton comprenant une huile de silicone volatile et un alcool gras à chaîne longue, un colorant physiologiquement acceptable hydrosoluble étant aussi intimement associé à l'antitranspirant physiologiquement acceptable.

8. Composition selon la revendication 7, caractérisée en ce que le bâton renferme en outre une cire physiologiquement acceptable.

9. Composition selon la revendication 7 ou 8, caractérisée en ce que le bâton comprend des parties de différentes couleurs.

10. Composition selon la revendication 7 ou 8, caractérisée en ce qu'elle comprend d'environ 12 à environ 25% en poids de l'antitranspirant physiologiquement acceptable contenant, en association intime, un colorant hydrosoluble physiologiquement acceptable; d'environ 35 à environ 65% en poids de l'huile de silicone volatile; et d'environ 10 à environ 30% en poids d'un alcool gras de masse moléculaire élevée, la proportion de colorant étant d'environ 0,0002 à environ 0,1% du poids de la composition.

11. Composition selon la revendication 10, caractérisée en ce que l'alcool gras est l'alcool stéarylique.

12. Composition selon l'une quelconque des revendications 3 à 6, caractérisée en ce qu'elle convient pour être utilisée dans un distributeur à bille et en ce qu'elle comprend une huile de silicone volatile et dans laquelle est dispersé l'antitranspirant physiologiquement acceptable avec lequel est associé intimement un colorant hydrosoluble physiologiquement acceptable.

13. Composition selon la revendication 12, caractérisée en ce qu'elle comprend d'environ 12 à environ 25% en poids de l'antitranspirant physiologiquement acceptable, un colorant hydrosoluble physiologiquement acceptable étant intimement associé avec au moins une partie dudit antitranspirant, d'environ 2 à environ 25% en poids d'un agent gélifiant et d'environ 60 à environ 80% en poids de l'huile de silicone volatile, ledit colorant constituant d'environ 0,0002% à environ 0,1% du poids de la composition.

14. Bâton antitranspirant comprenant un sel antitranspirant astringent dispersé dans une matrice hydrophobe anhydre, caractérisé en ce que le bâton comporte des parties colorées et des parties sans couleur, un colorant hydrosoluble physiologiquement acceptable étant intimement associé avec l'antitranspirant de type sel astringent dans les parties colorées.

15. Procédé de préparation d'une composition colorée selon la revendication 1, caractérisé par les étapes qui consistent à mélanger une solution aqueuse d'un solide hydrosoluble et un colorant hydrosoluble, à éliminer l'eau du mélange résultant pour obtenir un solide coloré, à réduire le solide en une forme pulvérulente et à mélanger la poudre colorée avec le(s) autre(s) ingrédient(s) de ladite composition.

16. Procédé de préparation d'une composition antitranspirante colorée selon la revendication 3, caractérisé par les étapes qui consistent à mélanger une solution aqueuse de l'antitranspirant physiologiquement acceptable avec un colorant hydrosoluble physiologiquement acceptable, à éliminer l'eau du mélange résultant pour obtenir un produit sec, à réduire le produit en une forme pulvérulente et à mélanger la poudre avec le(s) ingrédient(s) de la matrice.

## Ansprüche

1. Gefärbte Zusammensetzung aus einer wasserfreien hydrophoben Matrix mit wachs-, gel-, creme- oder ölartiger Konsistenz und darin dispergiert einem wasserlöslichen Feststoff, dadurch gekennzeichnet, daß die Zusammensetzung mit einem wasserlöslichen, mit dem wasserlöslichen Feststoff innig verbundenen Farbstoff gefärbt ist.

2. Gefärbte kosmetische Arzneimittelzusammensetzung aus einer wasserfreien hydrophoben Matrix mit wachs-, gel-, creme- oder ölartiger Konsistenz und darin dispergiert einem wasserlöslichen physiologisch annehmbaren Feststoff, dadurch gekennzeichnet, daß die Zusammensetzung mit einem wasserlöslichen, physiologisch annehmbaren, mit dem wasserlöslichen physiologisch annehmbaren Feststoff innig verbundenen Farbstoff gefärbt ist.

3. Gefärbte Antiperspiranszusammensetzung aus einer wasserfreien hydrophoben Matrix und darin dispergiert einem physiologisch annehmbaren Antiperspirans, dadurch gekennzeichnet, daß die Zusammensetzung mit einem physiologisch annehmbaren, wasserlöslichen, mit dem physiologisch annehmbaren Antiperspirans innig verbundenen Farbstoff gefärbt ist.

4. Zusammensetzung nach Anspruch 3, dadurch gekennzeichnet, daß das Antiperspirans ausgewählt ist aus astringierenden Aluminium- und Aluminium-Zirkoniumsalzen.

5. Zusammensetzung nach Anspruch 4, dadurch gekennzeichnet, daß das Antiperspirans ausgewählt ist aus Aluminiumchlorhydrat, Aluminium-Zirkoniumtetrachlorhydrat-glyzinkomplex und Aluminiumchlorhydrat-propylen-glykolkomplex.

6. Zusammensetzung nach einem der Ansprüche 3 bis 5, dadurch gekennzeichnet, daß der Farbstoff in einem Gewichtsanteil von etwa 0,0002% bis etwa 0,1% bezüglich des Gewichts der Zusammensetzung vorhanden ist.

7. Zusammensetzung nach einem der Ansprüche 3 bis 6, dadurch gekennzeichnet, daß sie in der Form eines Stifts vorliegt aus einem flüchtigen Silikonöl und einem langkettigen Fettalkohol sowie dem physiologisch annehmbaren Antiperspirans, das einen innig damit verbundenen wasserlöslichen physiologisch annehmbaren Farbstoff aufweist.

8. Zusammensetzung nach Anspruch 7, dadurch gekennzeichnet, daß der Stift weiterhin ein physiologisch annehmbares Wachs enthält.

9. Zusammensetzung nach Anspruch 7 oder 8, dadurch gekennzeichnet, daß der Stift aus Teilen verschiedener Farbe besteht.

10. Zusammensetzung nach Anspruch 7 oder 8, dadurch gekennzeichnet, daß sie aus etwa 12 bis etwa 25 Gew.-% des physiologisch annehmbaren Antiperspirans, das einen innig damit verbundenen wasserlöslichen physiologisch annehmbaren Farbstoff aufweist; etwa 35 bis etwa 65 Gew.-% des flüchtigen Silikonöls; und etwa 10 bis etwa 30 Gew.-% eines hochmolekularen Fettalkohols besteht, wobei der Farbstoffanteil etwa 0,0002 bis etwa 0,1 Gew.-% der Zusammensetzung beträgt.

11. Zusammensetzung nach Anspruch 10, dadurch gekennzeichnet, daß es sich bei dem Fettalkohol um Stearylalkohol handelt.

12. Zusammensetzung nach einem der Ansprüche 3 bis 6, dadurch gekennzeichnet, daß sie zur Verwendung in einem Roller geeignet ist und aus flüchtigem Silikonöl und darin dispergiert dem physiologisch annehmbaren Antiperspirans das einen damit innig verbundenem physiologisch annehmbaren wasserlöslichen Farbstoff aufweist, besteht.

13. Zusammensetzung nach Anspruch 12, dadurch gekennzeichnet, daß sie aus etwa 12 bis etwa 25 Gew.-% des physiologisch annehmbaren Antiperspirans, wobei zumindest ein Teil jenes Antiperspirans einen wasserlöslichen physiologisch annehmbaren damit innig verbundenen Farbstoff aufweist, etwa 2 bis etwa 25 Gew.-% eines Geliermittels und etwa 60 bis etwa 80 Gew.-% des flüchtigen Silikonöls besteht, wobei jener Farbstoff etwa 0,0002 bis etwa 0,1 Gew.-% der Zusammensetzung enthält.

14. Antiperspiransstift aus einem astringierenden in einer wasserfreien hydrophoben Matrix dispergierten Antiperspiranssalz, dadurch gekennzeichnet, daß der Stift gefärbte Teile und ungefärbte Teile aufweist, wobei das astringierende Salzantiperspirans in den gefärbten Teilen einen innig damit verbundenen physiologisch annehmbaren wasserlöslichen Farbstoff aufweist.

15. Verfahren zur Herstellung einer gefärbten Zusammensetzung nach Anspruch 1, gekennzeichnet durch die Schritte: Mischen einer wässrigen Lösung eines wasserlöslichen Feststoffes und eines wasserlöslichen Farbstoffes, Entfernen des Wassers aus der resultierenden Mischung unter Bildung eines gefärbten Feststoffes, Zerkleinerung des Feststoffes zu einem Pulver und Mischen des gefärbten Pulvers mit dem(den) übrigen Bestandteil(en) jener Zusammensetzung.

16. Verfahren zur Herstellung einer gefärbten Antiperspiranszusammensetzung nach Anspruch 3, gekennzeichnet durch die Schritte: Mischen einer wässrigen Lösung des physiologisch annehmbaren Antiperspirans mit einem wasserlöslichen physiologisch annehmbaren Farbstoff, Entfernen des Wassers aus der resultierenden Mischung unter Bildung eines trockenen Produktes, Zerkleinerung des Produktes zu einem Pulver und Mischen des Pulvers mit dem (den) Matrixbestandteil(en).